# EUROPÄISCHE PATENTSCHRIFT

(11) **EP 0 815 789 B1**
(45) Veröffentlichungstag und Bekanntmachung des Hinweises auf die Patenterteilung: **31.03.2004**
(21) Anmeldenummer: 97107274.9
(22) Anmeldetag: 02.05.1997
(51) Int. Cl.: A61B 3/024, A61B 3/06

(54) **Gerät zur Prüfung des Gesichtsfeldes des menschlichen Auges**
Apparatus for testing the visual field of the human eye
Appareil d'examen du champ visuel de l'oeil humain

(30) Priorität: 24.06.1996 DE 19625199
(43) Veröffentlichungstag der Anmeldung: 07.01.1998
(60) Teilanmeldung: 01114093.6
(73) Patentinhaber: Oculus Optikgeräte GmbH, 35582 Wetzlar (DE)
(72) Erfinder: Köst, Gert, Diplom-Biologe, 30171 Hannover (DE)
(74) Vertreter: Böck, Bernhard, Dipl.-Ing.

(56) Entgegenhaltungen:
- CH-A- 264 664
- FR-A- 2 301 215
- US-A- 2 803 990
- US-A- 2 837 964
- US-A- 3 947 099
- US-A- 4 561 738

## Beschreibung

Die Erfindung betrifft ein Gerät zur Prüfung des Gesichtsfeldes des menschlichen Auges mit einem schalenförmigen Schirm, auf dem eine oder mehrere Testmarken projiziert werden, wobei der Schirm aus einem transparenten Material besteht, die Lichtquelle für die Testmarke auf einem der Kontur des Schirmes mit äquidistantem Abstand folgenden und auf dessen Rückseite liegenden Schwenkarm angeordnet ist, die Testmarke von der Rückseite her auf den Schirm etwa senkrecht zur Flächennormalen projiziert wird, und die Lichtquelle und damit die Testmarke flächig über dessen Rückseite verfahrbar angeordnet ist.

In der augenärztlichen Praxis spielt die Messung des Gesichtsfeldes neben der Druckmessung eine wesentliche Rolle. Bekannt ist es, zur Messung des Gesichtsfeldes das Perimeter von Goldmann einzusetzen, das es gestattet, eine kinetische-quantitative wie auch eine statisch-quantitative Untersuchung durchzuführen. Diese kinetisch-quantitative Perimetrie arbeitet mit einer beweglichen Testmarke bestimmter Leuchtdichte, mit der man die Stellen gleicher Unterschiedsempfindlichkeit senkrecht auf die zu erwartenden Gesichtsfeldgrenzen bzw. Isopteren aufsucht, was durch die freie Beweglichkeit der Testmarke in jeder Richtung gewährleistet wird. Bei der statisch-quantitativen Perimetrie wird dem Probanden eine feststehende Testmarke mit bestimmter Leuchtdichte angeboten, die an einer beliebigen Stelle im Gesichtsfeld als überschwellig erkannt wird. Beide Untersuchungsmethoden werden in der Regel nebeneinander durchgeführt, so daß ein Gerät vorteilhaft ist, mit dem beide Untersuchungsmethoden durchgeführt werden können.

Das Goldmann-Perimeter ist ein schalenförmiges Projektionsperimeter mit direkter Registrierung der Testmarkenposition. Die gleichmäßige und konstante Ausleuchtung der Innenseite der Schale soll bei jeder Untersuchung stets den gleichen Adaptionszustand des Auges gewährleisten. Die Testmarke, mit welcher man die Reizschwelle des Lichtsinnes in den zentralen und peripheren Gesichtsfeldpartien bestimmt, wird auf den Grund der Schale von der Schalenöffnung her projiziert. Da jedoch der Probandenkopf genau auf das Zentrum der Schale fixiert ist, ist es zwangsnotwendig, daß das Projektionssystem exzentrisch angeordnet ist. Durch diese exzentrische Anordnung ergeben sich unterschiedlich starke Verzeichnungen der Testmarke in Abhängigkeit der jeweils gewählten Testmarkenposition. Hierdurch ändert sich nicht nur die Form der Testmarke, sondern auch deren Helligkeitsverteilung, so daß hierdurch Fehlerquellen auftreten können.

Das Dokument CH-A-264 664 offenbart ein Gerät zur Prüfung des Gesichtsfeldes des menschlichen Auges mit den Merkmalen des Oberbegriffs des Anspruchs 1.

Die Erfindung ist in den angefügten Ansprüchen definiert.

Der Erfindung liegt die Aufgabe zugrunde, ein Gerät der eingangs genannten Art so auszubilden, daß dieses zum einen für die statische wie auch für die kinetische Perimetrie geeignet ist, das es gestattet, Testmarken gleichmäßiger Größe und gleichmäßiger Helligkeit im gesamten Prüfbereich anzubieten und das darüber hinaus einfach im Aufbau und in der Betriebsweise ist und weiterhin eine Prüfung der Farbtüchtigkeit zuläßt.

Diese Aufgabe wird mit den Merkmalen des Anspruches 1 gelöst.

Ein erfindungsgemäßes Gerät besteht somit aus einem Schirm aus einem transparenten Material, auf dessen Rückseite die Testmarke in etwa senkrecht zur Flächennormale projiziert wird, wobei die Lichtquelle und damit die Testmarke flächig über die Rückseite des Schirmes verfahrbar angeordnet ist, wobei der Schirm aus einem das Licht dämpfenden Material besteht. Das Verfahren der Lichtquelle längs der Rückseite des Schirmes erfolgt in etwa einem äquidistanten Abstand zur Oberfläche des Schirmes, so daß die Testmarke stets die gleiche Größe aufweist und durch die Projektion der Testmarke zur Normalen des Schirmes auch überall stets die gleiche Helligkeit hat.

Dadurch, daß der Schirm des Gerätes aus einem lichtdämpfenden Material besteht, erreicht man den Vorteil, daß an der Innenfläche des Schirmes reflektiertes Licht, das eine große optische Weglänge innerhalb des Materials des Schirmes zurücklegt, ehe es wieder auf die Sichtseite des Schirmes gelangt, so weit gedämpft wird, daß es vom Probanden nicht mehr wahrgenommen werden kann.

Vorteilhaft besteht die Lichtquelle für die Testmarken aus einer oder mehreren LED's oder aber Laserdioden, deren Licht in einem bestimmten Verhältnis gemischt wird. Hierdurch ist es möglich, dem Probanden eine weiße oder aber eine beliebige farbige Testmarke anzubieten. Für die Homogenisierung des Lichtes wird vorteilhaft ein Glasstab eingesetzt, auf dessen einer Seite die Dioden angeordnet sind. Vorteilhaft ist zwischen der Abbildungsoptik und der Lichtquelle ein halbdurchlässiger Spiegel angeordnet, über den ein reflektierter Strahl ausgeblendet werden kann, der z. B. zur automatischen Feststellung und Regulierung der Helligkeit herangezogen werden kann.

Eine vorteilhafte Ausbildung eines erfindungsgemäßen Gerätes besteht darin, daß eine oder mehrere Lichtquellen auf einem Schwenkarm angeordnet sind, der entsprechend der Krümmung des Schirmes ausgebildet ist. Dieser Schwenkarm ist um eine Achse drehbar, die senkrecht zur Projektionsfläche des Schirmes verläuft. Die Länge des Schwenkarmes muß mindestens so groß sein, daß dieser bis zum Rand des Schirmes reicht, so daß beim Drehen des Schwenkarmes dieser die gesamte Schirmfläche überstreicht. Die zur Erzeugung der Testmarken erforderlichen Lichtquellen sind auf dem Schwenkarm angeordnet, wobei diese fest oder aber auch beweglich auf dem Schwenkarm angebracht sein können. Vorteilhaft sind die Lichtquellen fest auf dem Schwenkarm angeordnet, wobei der Schwenkarm als solcher relativ zu seiner Längsachse verschiebbar ist, so daß die Lichtquellen mit ihrer Abbildungsoptik zum einen über den halben Umfang des Schirmes verschiebbar sind und durch Drehen des Schwenkarmes um seine Schwenkachse auf jede beliebige Stelle der Oberfläche des Schirmes gelangen können. Der Abstand der Lichtquellen und der Projektionsoptik von der Oberfläche des Schirmes ändert sich bei der Bewegung der Lichtquellen nicht.

Gemäß einer vorteilhaften Ausführungsform ist der Schwenkarm mittels dreier Führungsrollen an einer an der Drehachse befestigten Montageplatte gehalten, wobei diese Führungsrollen an gegenüberliegenden Seiten des Schwenkarmes angreifen und jeweils einen Abstand zueinander aufweisen. Hierdurch kann zum einen der Schwenkarm relativ zur Achse verschoben werden und behält zum anderen seine Lage relativ zum Schirm stets bei.

Als Antriebsmittel hat sich ein Zahnriemen bewährt, der an den Enden des Schwenkarmes befestigt ist und im Bereich eines Antriebsritzels von dem Schwenkarm über Umlenkrollen angehoben wird.

Weitere vorteilhafte Ausgestaltungen der Erfindung gehen aus der Beschreibung und den Ansprüchen hervor.

Ein Ausführungsbeispiel der Erfindung ist im folgenden anhand der Zeichnungen näher beschrieben. In diesen zeigen:
- Fig. 1: einen Schnitt durch ein erfindungsgemäß ausgebildetes Gerät,

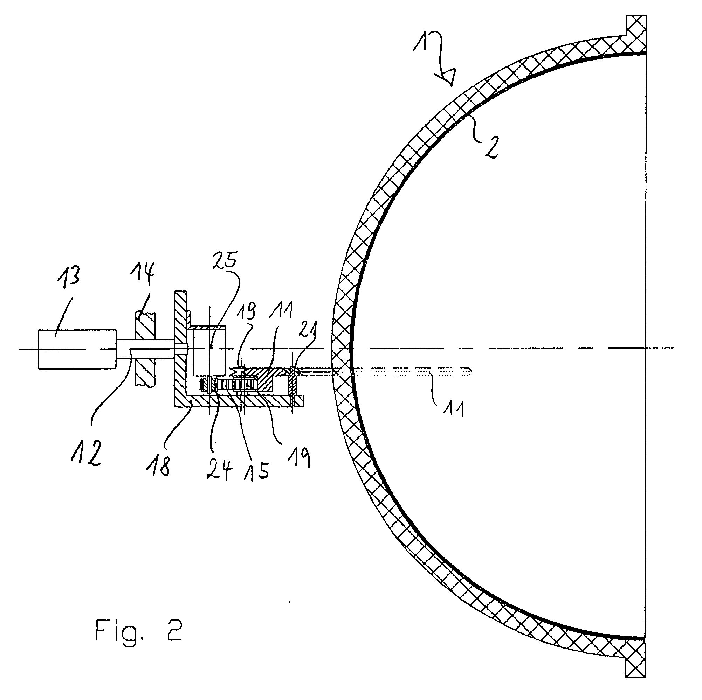

- Fig. 2: einen um 90 ° hierzu verlaufenden Schnitt und
- Fig. 3: die Einzelheit X in Fig. 1 in vergrößertem Maßstab.

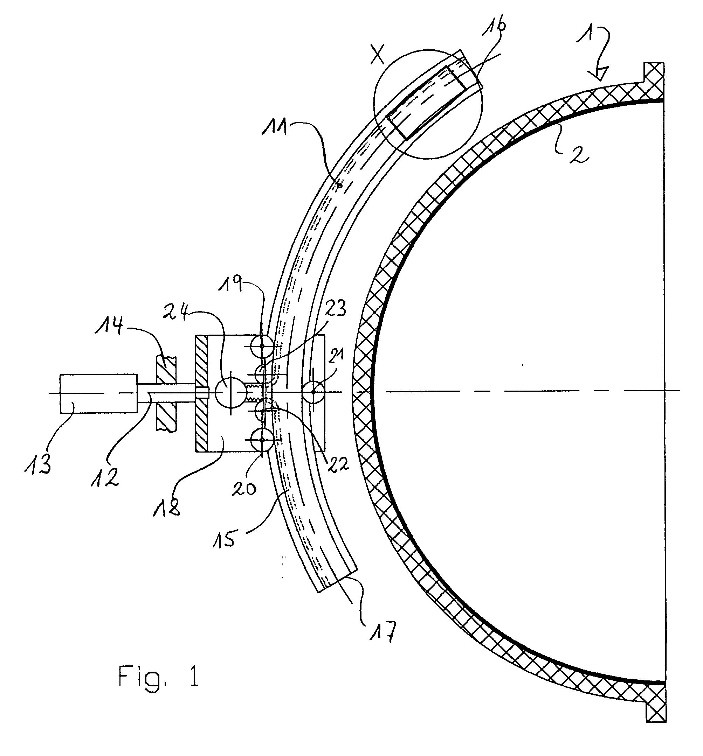

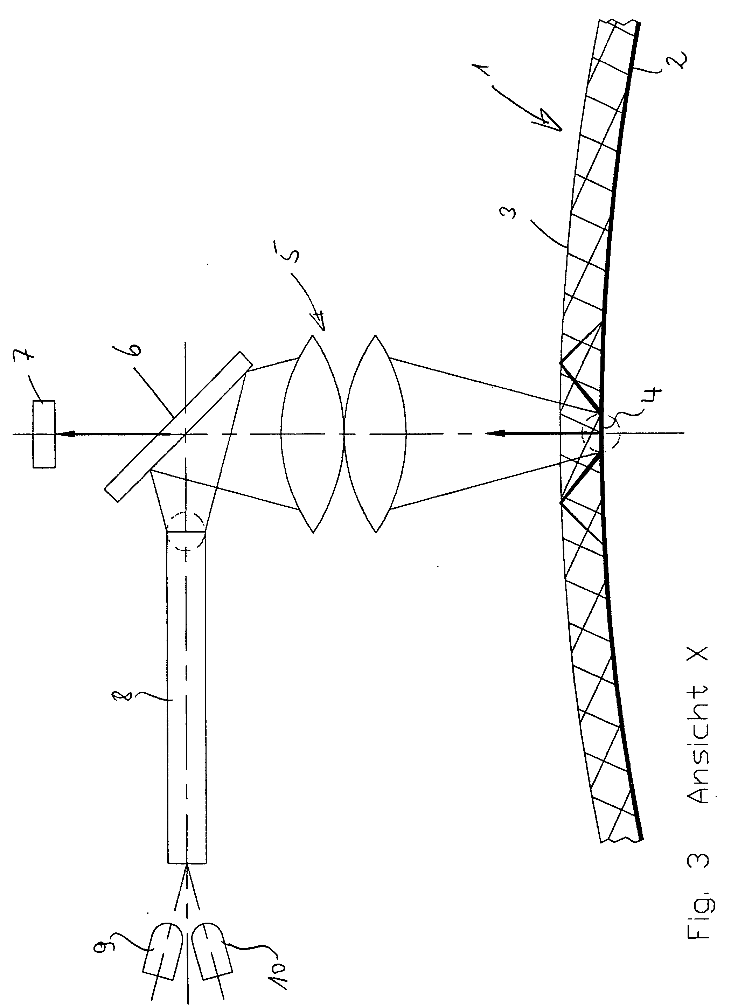

In Fig. 1 ist ein Horizontalschnitt durch ein erfindungsgemäßes Perimeter dargestellt, wobei der Schirm 1 halbkugelförmig ausgebildet ist und auf seiner Innenfläche eine Projektionsfläche 2 aufweist. Der Proband sieht mittig in den Schirm hinein, wobei sein Auge durch eine im Ausführungsbeispiel nicht dargestellte Fixationsmarke fixiert ist. Der Schirm 1 besteht aus einem durchsichtigen oder durchscheinenden Material, wobei dieses Material eine relativ große Lichtdämpfung aufweist, so daß, wie aus der Fig. 3 ersichtlich ist, die von der Testmarke 4 reflektierten Strahlen bis zur Rückseite 3, an der sie wiederum reflektiert werden, bereits in ihrer Helligkeit wesentlich gedämpft sind, so daß diese, wenn sie wiederum auf die Vorderseite, d. h. auf die Projektionsfläche 2 des Schirmes auftreffen, vom Auge des Probanden nicht mehr wahrgenommen werden. Hierdurch wird vermieden, daß der Proband kreisförmige Ringe um die Testmarke sieht.

Zur Erzeugung der Testmarke 4 auf der Projektionsfläche 2 des Schirmes 1 dienen zwei Dioden 9, 10, die ihr Licht auf einen Glasstab 8 abgeben. Von diesem Glasstab aus trifft das Licht auf einen halbdurchlässigen Spiegel 6, der unter 45 ° in dem Strahlengang angeordnet ist. Von diesem Spiegel aus wird das Licht zum Schirm 1 über eine Abbildungsoptik 5 geleitet, die eine exakt definierte Testmarke 4 erzeugt. Das von der Testmarke 4 zurückgeworfene Licht erreicht durch den halbdurchlässigen Spiegel 6 eine Fotozelle 7, mit der die Helligkeit des reflektierten Lichtes gemessen wird. Das Meßsignal wird zur Ansteuerung der Dioden 9, 10 herangezogen, so daß die Testmarke 4 in ihrer Helligkeit auf vorgegebene Werte exakt einregelbar ist.

Die gesamte Abbildungsoptik 5, der halbdurchlässige Spiegel 6, die Fotozelle 7, der Glasstab 8, der zur Homogenisierung des Lichtes der Dioden 9, 10 dient, und die Dioden 9, 10 sind auf einem Schwenkarm 11 befestigt, der an einer Montageplatte 18 befestigt ist. Die Halterung des Schwenkarmes 11 erfolgt über drei Führungsrollen 19, 20, 21, die jeweils auf einer Seite des Schwenkarmes 11 angeordnet sind. Wird der Schwenkarm 11, der der Kontur der Schüssel derart angepaßt ist, daß dieser in allen seinen Bereichen einen äquidistanten Abstand zur Projektionsfläche 2 des Schirmes 1 aufweist, verschoben, so hat die Lichtquelle zur Erzeugung der Testmarke 4 immer den gleichen Abstand und Ausrichtung zur Testfläche. Der Schwenkarm 11 kann innerhalb der Führungsrollen 19, 20, 21 verschoben werden, so daß eine fest auf dem Schwenkarm 11 angeordnete Lichtquelleneinheit 9, 10 parallel zur Schirmoberfläche verschoben wird.

Um eine einfache Verschiebung des Schwenkarmes 11 relativ zu seinen Führungsrollen 19, 20 und 21 zu erhalten, ist an dessen Enden 16, 17 ein Zahnriemen 15 befestigt, der über zwei Umlenkrollen 22, 23 von dem Schwenkarm 11 abgehoben wird und über ein Antriebszahnrad 24 geführt wird. Dieses Zahnrad wird von einem Motor 25 angetrieben.

Der Schwenkarm 11 seinerseits ist auf einer Montageplatte 18 befestigt, an der auch die Führungsrollen 19, 20, 21, die Umlenkrollen 22, 23 sowie das Antriebszahnrad 24 befestigt sind. Diese Montageplatte wiederum ist an einer Achse 12 befestigt, die am Rahmen 14 des Gerätes befestigt ist. Diese Achse ist senkrecht zur Projektionsfläche 2 des Schirmes angeordnet, d. h. die Verlängerung der Achse verläuft durch den Mittelpunkt des Schirmes. Wird diese Achse 12 durch den Motor 13 gedreht, so wird die Lichtquelle 9, 10 und damit auch die Testmarke 4 auf einem Kreis bewegt. Durch Verdrehen wie auch durch Verschieben des Schwenkarmes 11 relativ zur Montageplatte 18 und damit zur Achse 12 kann an jeder beliebigen Stelle der Projektionsfläche 2 des Schirmes 1 eine Testmarke erzeugt werden.

Im Ausführungsbeispiel ist lediglich eine Lichtquelle dargestellt, es ist jedoch auch möglich, mehrere Lichtquellen auf dem Schwenkarm anzuordnen, so daß auch Lichtpunkte an unterschiedlichen Stellen des Schirmes ohne eine Verschiebung der Lichtquelle oder aber ein Verdrehen der Achse erzeugt werden können.

Die Lichtquelle 9, 10 besteht vorteilhaft aus zwei oder drei Dioden, wobei hier entweder bei der Verwendung von zwei Dioden blaue und gelbe Dioden eingesetzt werden oder aber bei der Verwendung von drei Dioden gelbe, rote und grüne Dioden. Zur Erhöhung der Leuchtdichte kann die Anzahl der Dioden auch vervielfacht werden, oder aber es werden Laserdioden eingesetzt, die eine erhöhte Lichtenergie abgeben können. Der Vorteil der Verwendung von Dioden besteht darin, daß durch Mischen des Lichtes der Dioden eine weiße Testmarke oder aber eine beliebige farbige Testmarke auf der Projektionsfläche erzeugt werden kann. Es sind somit Farbuntersuchungen, z. B. Blau-Perimetrie, möglich. Mit der Blau-Perimetrie und einem gelben Umfeld lassen sich verschiedene Krankheitsbilder, wie z. B. Glaukome, diabetische Retinopathie, früher erkennen, als dies bei einer weißen Testmarke der Fall ist.

Als Projektionssystem wird bevorzugt ein gealtertes Diodensystem verwandt. Helligkeitsänderungen werden vorteilhaft per Frequenzzeit-Modulation realisiert. Des weiteren ist es ohne weiteres möglich, mit dem erfindungsgemäßen Gerät unterschiedliche Testmarkengrößen anzubieten, wobei übliche Testmarkengrößen 10 Winkelminuten oder 30 Winkelminuten aufweisen.

Die Fixation des Auges kann in üblicher Weise durch eine fest installierte Fixationsmarke erreicht werden, oder aber es wird über eine CCD-Kamera direkt ein Segmentbild eingeblendet, wobei mit einer nicht dargestellten einstellbaren Kinnstütze das zu untersuchende Auge exakt auf die Kugelmitte positioniert werden kann.

Des weiteren ist es durchaus möglich, an beliebigen weiteren Stellen zusätzliche Fixationsmarken anzuordnen.

Alternativ zum Dioden-Projektionssystem könnte auch eine Lichtfaserleitung eingesetzt werden, die von einer zentral plazierten Lichtquelle gespeist wird. Zwischen der Lichtquelle und dem Lichtleiter kann ein Filtersystem oder ein Graukeil eingeschaltet werden, um eine entsprechende Helligkeitsabstufung in einfacher Weise zu erhalten. Des weiteren kann ein Verschlußsystem vorgesehen werden, um die Testmarkendarstellung in zeitlichen Abständen anbieten zu können.

Mit einem erfindungsgemäßen Gerät können sowohl kinetische wie auch statische Augenuntersuchungen auch in der Weise, wie dies mit dem Goldmann-Perimeter der Fall ist, durchgeführt werden, so daß die mit dem erfindungsgemäßen Perimeter aufgefundenen Werte exakt mit den mit dem Goldmann-Perimeter aufgefundenen vergleichbar sind.

## Patentansprüche

1. Gerät zur Prüfung des Gesichtsfeldes des menschlichen Auges mit einem schalenförmigen Schirm, auf dessen Grund eine oder mehrere Testmarken projiziert werden, wobei, die Lichtquelle (9, 10) bzw. Lichtquellen für die Testmarke (4) bzw. Testmarken auf einem der Kontur des Schirmes (1) mit äquidistantem Abstand folgenden und auf dessen Rückseite liegenden Schwenkarm (11) angeordnet ist bzw. sind, die Testmarke (4) bzw. Testmarken von der Rückseite (3) her auf den Schirm (1) etwa senkrecht zur Flächennormalen projiziert wird bzw. werden, und die Lichtquelle bzw. Lichtquellen und damit die Testmarke (4) bzw. Testmarken flächig über dessen Rückseite verfahrbar angeordnet ist bzw. sind, wobei der Schirm (1) aus einem durchsichtigen oder durchscheinenden Material besteht,
**dadurch gekennzeichnet, daß** der einmal an der Projektionsfläche (2) des Schirms (1) reflektierte und danach einmal an der Rückseite (3) des Schirms reflektierte Lichtstrahl in der Helligkeit durch das Schirmmaterial so weit gedämpft wird, dass der Lichtstrahl danach an der Projektionsfläche (2) nicht mehr wahrgenommen werden kann.

2. Gerät nach Anspruch 1, **dadurch gekennzeichnet, daß** als Lichtquelle (9, 10) für die Testmarke (4) eine oder mehrere LED's oder Laserdioden dienen.

3. Gerät nach Anspruch 2, **dadurch gekennzeichnet, daß** entweder blaue und gelbe Dioden oder gelbe, rote und grüne Dioden eingesetzt werden, die jeweils eine Testmarke (4) beleuchten.

4. Gerät nach Anspruch 2 oder 3, **dadurch gekennzeichnet, daß** zur Mischung des von den Dioden (9, 10) ausgehenden Lichtes ein Glasstab (8) dient.

5. Gerät nach Anspruch 4, **dadurch gekennzeichnet, daß** der Glasstab (8) einen Durchmesser von ca. 2 mm aufweist.

6. Gerät nach einem der Ansprüche 1 bis 5, **dadurch gekennzeichnet, daß** der Schwenkarm (11) um eine Achse (12) drehbar ist, die senkrecht zur Projektionsfläche (2) des Schirmes (1) verläuft.

7. Gerät nach Anspruch 6, **dadurch gekennzeichnet, daß** der Schwenkarm (11) parallel zur Projektionsfläche (2) des Schirmes (1) verschiebbar ist.

8. Gerät nach Anspruch 6, **dadurch gekennzeichnet, daß** die Lichtquelle (9, 10) auf dem Schwenkarm (11) verschiebbar ist.

9. Gerät nach Anspruch 7 oder 8, **dadurch gekennzeichnet, daß** der Schwenkarm (11) durch mindestens drei Führungsrollen (19, 20, 21) geführt ist, die auf einer mit der Drehachse (12) verbundenen Montageplatte (18) befestigt sind.

10. Gerät nach Anspruch 9, **dadurch gekennzeichnet, daß** auf dem Schwenkarm (11) ein Zahnriemen (15) angeordnet ist, der an dem jeweiligen Ende (16, 17) des Schwenkarmes (11) befestigt ist, und daß der Zahnriemen (15) über zwei Umlenkrollen (22, 23) und ein Antriebszahnrad (24) geführt ist.

11. Gerät nach einem der Ansprüche 1 bis 10, **dadurch gekennzeichnet, daß** der Schirm (1) auf seiner Innenseite beschichtet ist.

12. Gerät nach einem der Ansprüche 2 bis 5, **dadurch gekennzeichnet, daß** die Dioden (9, 10) zur Erzeugung des Mischlichtes einzeln ansteuerbar ausgebildet sind.

13. Gerät nach einem der Ansprüche 1 bis 12, **dadurch gekennzeichnet, daß** eine oder mehrere Fixiermarken in dem Schirm (1) vorgesehen sind.

14. Gerät nach einem der Ansprüche 1 bis 13, **dadurch gekennzeichnet, daß** in dem Projektionsstrahl für die Testmarke (4) ein teildurchlässiger Spiegel (6) angeordnet ist, und daß der ausgeblendete Strahlenanteil in seiner Lichtstärke erfaßt werden kann und z. B. zur Steuerung der Helligkeit der Testmarke (4) dient.

15. Gerät nach Anspruch 14, **dadurch gekennzeichnet, daß** der teildurchlässige Spiegel (6) auf dem Schwenkarm (11) angeordnet ist.

## Claims

1. Apparatus for testing the visual field of the human eye with a dish-shaped screen on to the base of which one or more test marks are projected, wherein the light source (9, 10) or light sources for the test mark (4) or test marks is or are arranged on a swivel arm (11) which follows the contour of the screen (1) with an equidistant separation and lies on the reverse side thereof, the test mark (4) or test marks is or are projected from the reverse side (3) on to the screen (1) approximately perpendicular to the normal to the surface, and the light source or light sources and therefore the test mark (4) or test marks is or are arranged such that it or they can be moved in a planar manner over the reverse side thereof, the screen (1) being made of a transparent or translucent material, **characterized in that** the light beam, once reflected on the projection surface (2) of the screen (1) and then once reflected on the reverse side (3) of the screen, is attenuated in brightness by the screen material to the extent that the light beam can then no longer be perceived on the projection surface (2).

2. Apparatus according to claim 1, **characterized in that** one or more LEDs or laser diodes are used as the light source (9, 10) for the test mark (4).

3. Apparatus according to claim 2, **characterized in that** either blue and yellow diodes or yellow, red and green diodes, which each illuminate a test mark (4), are employed.

4. Apparatus according to claim 2 or 3, **characterized in that** a glass rod (8) is used for mixing the light emitted from the diodes (9, 10).

5. Apparatus according to claim 4, **characterized in that** the glass rod (8) has a diameter of approx. 2 mm.

6. Apparatus according to one of claims 1 to 5, **characterized in that** the swivel arm (11) is rotatable about an axis (12) which runs perpendicular to the projection surface (2) of the screen (1).

7. Apparatus according to claim 6, **characterized in that** the swivel arm (11) can be displaced parallel to the projection surface (2) of the screen (1).

8. Apparatus according to claim 6, **characterized in that** the light source (9, 10) can be displaced on the swivel arm (11).

9. Apparatus according to claim 7 or 8, **characterized in that** the swivel arm (11) is guided through at least three guide rolls (19, 20, 21) which are fixed to a mounting plate joined to the axis of rotation (12).

10. Apparatus according to claim 9, **characterized in that** on the swivel arm (11) there is arranged a toothed belt (15) which is fixed to the particular end (16, 17) of the swivel arm (11), and **in that** the toothed belt (15) is guided over two deflecting rolls (22, 23) and a driving toothed wheel (24).

11. Apparatus according to one of claims 1 to 10, **characterized in that** the screen (1) is coated on its inner side.

12. Apparatus according to one of claims 2 to 5, **characterized in that** the diodes (9, 10) for generation of the mixed light are constructed such that they can be controlled individually.

13. Apparatus according to one of claims 1 to 12, **characterized in that** one or more fixing marks are provided in the screen (1).

14. Apparatus according to one of claims 1 to 13, **characterized in that** a partly transparent mirror (6) is arranged in the projection beam for the test mark (4), and **in that** the light intensity of the blocked-out beam content can be detected and is used e.g. to control the brightness of the test mark (4).

15. Apparatus according to claim 14, **characterized in that** the partly transparent mirror (6) is arranged on the swivel arm (11).

## Revendications

1. Appareil pour l'examen du champ visuel de l'oeil humain, comprenant un écran en forme de coque sur la base duquel sont projetées une ou plusieurs marques de test, la source de lumière ou les sources de lumière (9, 10) pour la marque de test (4) ou les marques de test étant disposée(s) sur un bras pivotant (11) suivant le contour de l'écran (1) de façon équidistante et situé au niveau de son côté arrière, la marque de test (4) ou les marques de test étant projetée(s) depuis le côté arrière (3) sur l'écran (1) approximativement perpendiculairement à la normale à la surface et la source de lumière ou les sources de lumière et de ce fait la marque de test (4) ou les marques de test étant disposée(s) de manière à être déplaçables à plat sur son côté arrière,
l'écran (1) se composant d'un matériau transparent ou translucide,
**caractérisé en ce que** la luminosité du faisceau lumineux réfléchi une fois sur la surface de projection (2) de l'écran (1) et ensuite une fois sur le côté arrière (3) de l'écran est atténuée par le matériau de l'écran dans une mesure telle que le faisceau lumineux ne puisse ensuite plus être perçu sur la surface de projection (2).

2. Appareil selon la revendication 1, **caractérisé en ce que** l'on utilise comme source de lumière (9, 10) pour la marque de test (4) une ou plusieurs DEL ou diodes laser.

3. Appareil selon la revendication 2, **caractérisé en ce que** l'on utilise des diodes bleues et jaunes ou bien des diodes jaunes, rouges et vertes, qui éclairent chacune une marque de test (4).

4. Appareil selon la revendication 2 ou 3, **caractérisé en ce que** l'on utilise une barre de verre (8) pour combiner la lumière sortant des diodes (9, 10).

5. Appareil selon la revendication 4, **caractérisé en ce que** la barre de verre (8) présente un diamètre d'environ 2 mm.

6. Appareil selon l'une quelconque des revendications 1 à 5, **caractérisé en ce que** le bras pivotant (11) peut tourner autour d'un axe (12) qui s'étend perpendiculairement à la surface de projection (2) de l'écran (1).

7. Appareil selon la revendication 6, **caractérisé en ce que** le bras pivotant (11) peut être déplacé parallèlement à la surface de projection (2) de l'écran (1).

8. Appareil selon la revendication 6, **caractérisé en ce que** la source de lumière (9, 10) peut être déplacée sur le bras pivotant (11).

9. Appareil selon la revendication 7 ou 8, **caractérisé en ce que** le bras pivotant (11) est guidé par au moins trois galets de guidage (19, 20, 21) qui sont fixés sur une plaque de montage (18) liée à l'axe de rotation (12).

10. Appareil selon la revendication 9, **caractérisé en ce que** l'on dispose sur le bras pivotant (11) une courroie dentée (15) qui est fixée à chaque extrémité (16, 17) du bras pivotant (11) et **en ce que** la courroie dentée (15) est guidée par le biais de deux galets déflecteurs (22, 23) et d'une roue dentée d'entraînement (24).

11. Appareil selon l'une quelconque des revendications 1 à 10, **caractérisé en ce que** l'écran (1) est revêtu sur son côté interne.

12. Appareil selon l'une quelconque des revendications 2 à 5, **caractérisé en ce que** les diodes (9, 10) pour la production de la lumière composée sont réalisées de manière à pouvoir être commandées individuellement.

13. Appareil selon l'une quelconque des revendications 1 à 12, **caractérisé en ce que** l'on prévoit une ou plusieurs marques de fixation dans l'écran (1).

14. Appareil selon l'une quelconque des revendications 1 à 13, **caractérisé en ce que** l'on prévoit dans le faisceau de projection pour la marque de test (4) un miroir semi-argenté (6) et **en ce que** l'intensité lumineuse de la proportion de faisceau diaphragmée peut être détectée et sert par exemple à contrôler la luminosité de la marque de test (4).

15. Appareil selon la revendication 14, **caractérisé en ce que** le miroir semi-argenté (6) est disposé sur le bras pivotant (11).
